# EUROPEAN PATENT APPLICATION

(11) **EP 2 123 300 A1**
(43) Date of publication of application: **25.11.2009**
(21) Application number: 08724067.7
(22) Date of filing: 31.01.2008
(51) Int. Cl.: A61K 39/395, A61P 31/12

(54) **MEDICINAL AGENT FOR TREATING BIRD FLU**

(30) Priority: 02.02.2007 RU 2007103925
(71) Applicant: Dolgovykh, Lyudmila Fedorovna, Chelyabinsk 454091 (RU); Zilberman, Yakov Efimovich, Moscow 125413 (RU); Epshtein, Oleg Iliich, Moscow, 105064 (RU); Sergeeva, Svetlana Alexandrovna, Moscow 113535 (RU)
(72) Inventor: EPSHTEIN, Oleg Iliich, Moscow 105064 (RU); SERGEEVA, Svetlana Alexandrovna, Moscow 113535 (RU)
(74) Representative: Müller-Boré & Partner Patentanwälte
(86) International application number: PCT/RU2008/000056
(87) International publication number: WO 2008/097132

(57) **Abstract**

The inventive medicinal agent for treating bird flu comprises an antibody form which is activated and potentiated to alpha, beta or gamma bird interferon and which is obtainable by repeatedly and consecutively diluting and exposing it to an external action according to homeopathic technology providing an ultra-small antibody dose.

## Description

### Field of the Invention

The invention relates to the field of medicine, and specifically, to veterinary medicine, and can be used for effective treatment and prevention of avian influenza in poultry, and predominantly, chickens.

### Prior art

As known from the prior art, multivalent sera, containing antibodies to pathogens, causing specific diseases, are used as medicinal agents for treatment and prevention of viral infections in domestic animals (Lipin A.V., Sanin A.V., Zinchenko Y.V. VETERINARIAN REFERENCE BOOK. Traditional and non-traditional methods for treating dogs (in Rus.). Moscow, 2002, pp. 476-480). However, there are practically no specific medicinal agents for effective treatment and prevention of bird flu.

### Disclosure of the Invention

The invention is aimed at development of medicinal agent containing antibodies for effective treatment and prevention of avian flu in poultry, and predominantly, chickens.

The above objective is achieved by the fact that the medicinal agent for treatment of avian flu contains an activated and potentiated form of antibodies to avian alpha, beta or gamma interferon, obtained as a result of repeated successive dilution and exposure to an external action according to a homeopathic technology providing an ultra-small antibody dose.
Herein, the medicinal agent may contain a combination of activated and potentiated forms of antibodies to various avian alpha, beta or gamma interferons, or an activated and potentiated form of a mixture of various antibodies to avian alpha, beta or gamma interferons.

In addition, the medicinal agent contains a mixture of various homeopathic dilutions of antibodies to avian alpha, beta or gamma interferon in activated and potentiated form.

The medicinal agent, produced according to the invention, represents a new pharmacological drug containing antibodies, which is characterized by a specific pharmacological activity, sufficiently high efficacy and practically no side effects, which has been confirmed experimentally. Besides, the proposed medicinal agent is ecologically sound and of low cost.

### Embodiments of the Invention

The medicinal agent is prepared as follows.

In order to obtain antibodies to avian alpha, beta or gamma interferon, an immunogen, utilized for immunization of laboratory animals (rabbits), is used. Obtained antibodies were purified using affinity chromatography. A mixture of various fragments can also be used as immunogen.

A method of production of polyclonal immune and monoclonal antibodies is described, for example, in the book: "Immunological methods" (in Rus.)/edited by G. Frimel, Moscow, Meditsyna, 1987, pp. 9-33. A method of production of natural antibodies is described, for example, in the book: "Natural antibodies to low molecular weight compounds" (in Rus.). M.Ya. Myagkova, Moscow, MGUL, 2001 (ISBN 5-8135-0058-8), pp.70-114. A method of production of recombinant antibodies is described, for example, in the article: Laffly E., Sodoyer R. Hum Antibodies. Monoclonal and recombinant antibodies, 30 years after. - 2005 - Vol. 14. - No. 1-2, pp. 33-55.

Isolated antibodies are subject to successive dilutions and exposed to an external action, predominantly, vertical shaking, until the activated form is obtained in ultra-small doses (ultra-low doses), for example, by using a homeopathic technology of potentiation (see, for example, "Homeopathic medicinal agents. Production and description guide" (in Rus.), v. Shvabe, Moscow, 1967, pp. 12-38). In this case, the concentration is steadily reduced by successive dilution of 1 part by volume of the initial substance (antibodies) in 9 parts by volume (for decimal dilution D) or in 99 parts by volume (for centesimal dilution C) of a neutral solvent followed by multiple vertical shakings of each obtained dilution, at that it is preferable to use separate containers for each subsequent dilution - until a required dilution dose (potency) is obtained.

The external treatment in the process of concentration reduction can also be performed using ultrasonic, electromagnetic of other physical effect.

The medicinal agent prepared in such a way is predominantly used in pharmaceutical forms and dilutions, accepted in homeopathic practice, as alcohol or aqueous solutions or as tablets (granules) prepared by impregnating the neutral filler, contained within the pharmaceutical form, with the obtained solution - an activated antibody form - until saturation.

In order to increase curative (therapeutic) effect, the medicinal agent may also contain a mixture of various homeopathic dilutions of antibodies in activated and potentiated form.

### Example 1.

Leghorn layer hens, weighing 980 - 1030 g, from the experimental group consisting of 20 hens, respiratory infected with a lethal dose (3 LD₅₀) of avian flu (H5N1) virus (AFV), were given via free access an aqueous solution of ultra-small ( low) doses of activated and potentiated form of antibodies to avian gamma interferon - a mixture of homeopathic dilutions C12 + C30 + C200 (USD^{*} anti-IFN-gamma) (ULD of antibodies to IFN-gamma) - for 2 days before and 7 days after introduction of infection (in the control group consisting of 20 hens, infected hens were receiving distilled water in a similar mode).

As a result of infecting with a lethal dose of virus, hens in both groups died relatively quickly with not enough time for the clinical signs of the disease to develop. From 20 hens of the experimental group, 11 survived, while in the control group, only 5 hens survived (the percentage of survived hens in the experimental group was 2.2 times greater (p < 0.05) than that in the control group). On day 4 after introduction of infection, the AFV concentration in the lung tissues of hens of the control group was on the average 12% higher than in hens of the experimental group receiving a medicinal agent in the form of activated and potentiated antibodies to avian gamma interferon (ULD of antibodies to IFN- gamma).

Hence, when infecting poultry with the lethal dose of virus, a medicinal agent in the form of activated and potentiated antibodies to avian gamma interferon (ULD of antibodies to IFN-gamma).contributes to inhibiting the development of the infection process in lungs and reduces mortality among hens infected with avian flu virus.

### Example 2.

Leghorn layer hens, weighing 980 - 1030 g, from the experimental group consisting of 20 hens, respiratory infected with a lethal dose (3 LD₅₀) of avian flu (H5N1) virus (AFV), were given via free access an aqueous solution, containing a mixture of polyclonal antibodies to avian gamma interferon in activated and potentiated form (a mixture of homeopathic dilutions C12 + C30 + C200) and polyclonal antibodies to avian alpha interferon in activated and potentiated form (homeopathic dilution D50), for 2 days before and 7 days after introduction of infection (in the control group consisting of 20 hens, infected hens were receiving distilled water in a similar mode).

From 20 hens of the experimental group, 18 survived, while in the control group only 7 survived (the percentage of survived hens in the experimental group was 2.6 times greater (p < 0.05) than that in the control group). Clinical signs of the disease in both groups were observed only in those hens that died after being infected with the 3 LD₅₀ dose. The following external signs of the disease were registered in hens: 1) dormancy, lassitude; 2) photophobia; 3) impairment of coordination; 4) convulsions; and 5) meningeal symptoms at the moment of death. The number of clinical signs of the disease in hens from the experimental group receiving the medicinal agent, was 1.8 times less per 1 bird as compared to the control. On day 4 after introduction of infection, the AFV concentration in the lung tissues of hens of the control group was on the average 15% higher than in hens of the experimental group receiving the medicinal agent.

Hence, when infecting poultry with the lethal dose of virus, a medicinal agent in the form of a mixture of polyclonal antibodies to avian gamma interferon in activated and potentiated form (a mixture of homeopathic dilutions C12, C30 and C200) and polyclonal antibodies to avian alpha interferon in activated and potentiated form (homeopathic dilution D50) causes therapeutic and preventive effect expressed as its ability to increase the percentage of survived hens and inhibit reproduction of the virus in the lung tissues of birds.

### Example 3.

Leghorn layer hens, weighing 980 - 1030 g, from the experimental group consisting of 20 hens, respiratory infected with a lethal dose (3 LD₅₀) of avian flu (H5N1 strain) virus (AIV) (BFV), were given via free access an aqueous solution of ultra-low doses of activated and potentiated form of a mixture of antibodies to avian alpha and beta interferons in homeopathic dilution C30 (ULD of antibodies to antibodies to IFN-gamma) for 2 days before and 7 days after introduction of infection (in the control group consisting of 20 hens, infected hens were receiving distilled water in a similar mode).

From 20 hens of the experimental group, 19 survived, while in the control group only 7 survived. Clinical signs of the disease in both groups were observed only in those hens that died after being infected with the 3 LD₅₀ dose. The following external signs of the disease were registered in hens: 1) dormancy, lassitude; 2) photophobia; 3) impairment of coordination; 4) convulsions; and 5) meningeal symptoms at the moment of death. The number of clinical signs of the disease in hens from the experimental group receiving ULD of antibodies to IFN- gamma was 1.9 times less per 1 bird as compared to the control. On day 4 after introduction of infection, the AIV (BFV) concentration in the lung tissues of hens of the control group was on the average 15% higher than in hens of the experimental group receiving ULD of antibodies to IFN-gamma.

Hence, when infecting poultry with the avian flu virus, a medicinal agent in the form of activated and potentiated mixture of antibodies to avian alpha and beta interferons causes therapeutic (and prophylactic) effect, expressed as its ability to increase the percentage of survived hens (the percentage of survived hens in the experimental group was 2.7 times greater (p < 0.05) than that in the control group) and inhibit reproduction of the virus in the lung tissues of birds.

## Claims

1. A medicinal agent for treatment of avian flu, *wherein* said medicinal agent contains an activated and potentiated form of antibodies to avian alpha, beta or gamma interferon, obtained as a result of repeated successive dilution and exposure to an external action according to homeopathic technology providing an ultra- low dose of antibodies.

2. The medicinal agent of claim 1, *wherein* said medicinal agent contains a combination of activated and potentiated forms of antibodies to avian alpha, beta or gamma interferon.

3. The medicinal agent of claim 1, *wherein* said medicinal agent contains an activated and potentiated form of a mixture of various antibodies to avian alpha, beta or gamma interferon.

4. The medicinal agent of claim 1, *wherein* said medicinal agent contains a mixture of various homeopathic dilutions of antibodies to avian alpha, beta or gamma interferon in activated and potentiated form.
